# EUROPEAN PATENT SPECIFICATION

(11) **EP 4 105 339 B1**
(45) Date of publication and mention of the grant of the patent: **24.05.2023**
(21) Application number: 22178553.8
(22) Date of filing: 13.06.2022
(51) Int. Cl.: C12Q 1/6804

(54) **METHOD OF SPATIAL SEQUENCING OF GENES FROM TISSUE USING PADLOCKS WITH GAPS ON SUBSTRATE**
VERFAHREN ZUR RÄUMLICHEN SEQUENZIERUNG VON GENEN AUS GEWEBE UNTER VERWENDUNG VON PADLOCKS MIT LÜCKEN AUF EINEM SUBSTRAT
PROCÉDÉ DE SÉQUENÇAGE SPATIAL DE GÈNES À PARTIR DE TISSUS UTILISANT DES CADENAS AVEC DES INTERSTICES SUR LE SUBSTRAT

(30) Priority: 18.06.2021 EP 21180189
(43) Date of publication of application: 21.12.2022
(73) Proprietor: Miltenyi Biotec B.V. & Co. KG, 51429 Bergisch Gladbach (DE)
(72) Inventor: PINARD, Robert, 51429 Bergisch Gladbach (DE); MEYER, Hansueli, 51429 Bergisch Gladbach (DE)
(74) Representative: Kisters, Michael Marcus

(56) References cited:
- WO-A1-2016/162309
- WO-A1-2019/068880
- Xiaoyin Chen ET AL: "Efficient in situ barcode sequencing using padlock probe-based BaristaSeq", Nucleic Acids Research, vol. 46, no. 4, 28 November 2017 (2017-11-28), pages e22-e22, XP055751607, GB ISSN: 0305-1048, DOI: 10.1093/nar/gkx1206

## Description

### BACKGROUND

The invention is directed to a method for obtaining sequencing and spatial information of genes on a subcellular level on tissue.

It has been challenging to detect all expressed genes on a sub cellular level on tissue by keeping the spatial information and afterwards sequences those genes for potential variants. It is especially challenging to reach sub cellular resolution. The described method in this application allows a theoretical resolution down to a few tens of nanometers and allows to link the spatial information on the tissue with the sequenced information of the expressed gene.

Such technology is developed by the company Spatial Transcriptomics and 10X genomics. Here the spatial information is maintained as the tissue RNA molecules are tagged with a spatial identifier pre-spotted on an array. The resulting libraries are later sequenced by standard in vitro NGS sequencing approaches on Illumina for example. With the spotting process the position of the spatial identifier on the array is known before the sequencing process takes place. After sequencing of the spatial identifier, the linked RNA sequence of interest can be assigned to the tissue location. One major limitation of this approach is the resolution of the technology as it is dependent on the feature size of the spots on the array which is currently on a multicellular level only. Spatial Transcriptomics has multiple granted patents whereas the molecules are barcode labeled and are sequenced off substrate. It is also necessary to do a library preparation of all collected barcoded molecules. The presented method here does not remove the molecules from the substrate instead directly determines the sequence on the substrate. References are: US20150344942A1 METHODS AND PRODUCT FOR OPTIMISING LOCALISED OR SPATAL DETECTION OF GENE EXPRESSION IN A TISSUE; WO2016162309A1 SPATIALLY DISTINGUISHED, MULTIPLEX NUCLEIC ACID ANALYSIS OF BIOLOGICAL SPECIMENS and WO2019068880.

Another approach to combine genetic and special information are disclosed in WO2012/140224, by Fredrik Salmén et al in Nature protocols 2018 "Barcoded solid-phase RNA capture for Spatial Transcriptomics profiling in mammalian tissue"; Sanja Vickovic et al. "High-density spatial transcriptomics arrays for in situ tissue profiling", Nature methods, September 9 2019 and Xiaoyin Chen ET AL, "Efficient in situ barcode sequencing using padlock probe-based BaristaSeq", Nucleic Acids Research, GB, (20171128), vol. 46, no. 4, , pages e22 - e22.

### SUMMARY

The described method is being used to detect mRNA on tissue with a high spatial resolution of 300-500 nm with a simplified workflow compared to other competitive solutions in the market right now and without the requirement for a spatial identifier.

Object of the invention is therefore a method to obtain the spatial location and sequence information of a target sequence in a sample comprising at least one m-RNA strand comprising the steps
a. providing a surface with a plurality of spacer units capable of binding at least one m-RNA strand and with at least one fiducial marker
b. providing a sample comprising at least one m-RNA strand to the surface wherein at least one m-RNA strand of the sample binds to at least one spacer unit creating at least one single stranded oligomer
c. taking a first image of the surface to obtain the spatial information of the sample relative to the fiducial marker
d. removing sample form surface
e. hybridizing at least one oligonucleotide comprising 50 - 1000 nucleic acids with its 5' and 3' ends to complementary parts of the single stranded oligomer thereby forming a padlock-shaped structure that is ligated to create a single strand circular template
f. multiplying the single strand circular template by a polymerase capable of rolling circle amplification into a plurality of DNA concatemers thereby forming rolonies
g. obtaining the sequence information of the rolonies
h. linking the spatial information of the sample with the sequence information of the rolonies.

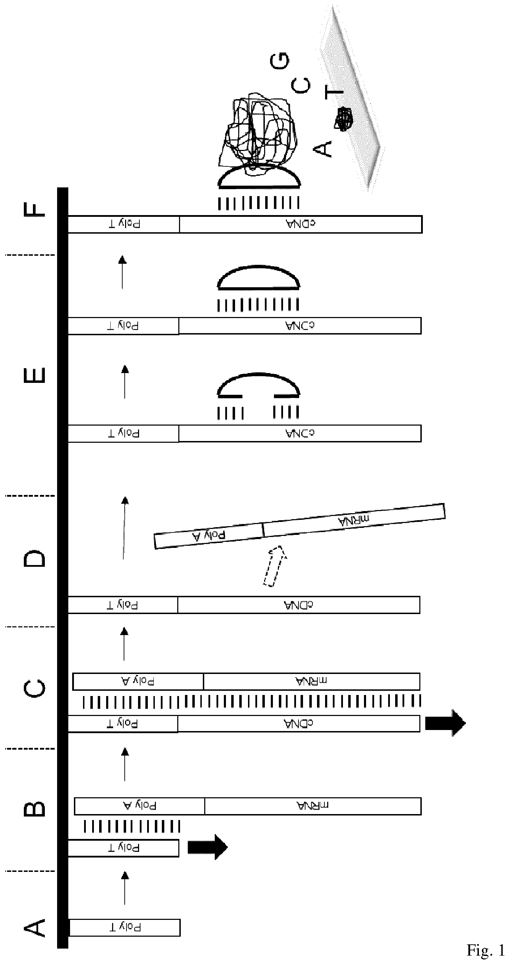

In a first embodiment of the invention as shown in Fig. 1 , the spacer units are selected from the group consisting of oligonucleotides comprising at least 5, preferable 5 to 50 Thymine single molecules (referred to as "poly-T"), wherein the single stranded oligomer bond to the at least one spacer unit is reverse transcribed into a c-DNA strand and wherein the mRNA strand is removed by denaturation.

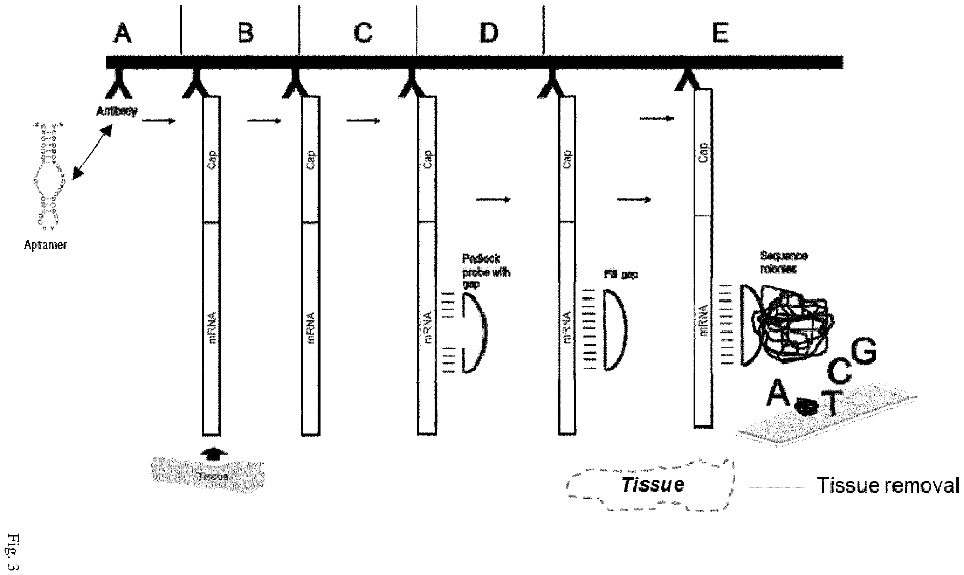

In a second embodiment of the invention as shown in Fig. 3, the spacer units are selected from the group consisting of antibodies, Fab fragments of antibodies, single chain Fv (scFv) fragments, divalent single chain antibodies or diabodies or aptamers. Preferable a recombinant human antibody (eIF4E) or a monoclonal antibody (Anti-7-methylguanosine (m7G) mAb) is used to target the cap-end of the mRNA on the 5' end.

### BRIEF DESCRIPTION OF THE DRAWINGS

Fig 1 shows a general workflow of the first embodiment of the invention
Fig 2 shows the spacial arrangement of the spacer units, fiducial markers and tissue on the surface of the invention.
Fig 3 shows a general workflow of the second embodiment of the invention

### DETAILLED DESCRIPTION

The method of the invention is conducted in several steps which are explained in detail in the following:

### Step a)

First, a solid, flat two-dimensional substrate is provided with a functionalized surface, for example with an activated carboxylate group or succinimidyl ester. To this activated surface, the spacer groups are attached. In the variant of poly-T groups, this can be achieved by amine-modified oligonucleotides; in another variant using antibodies provided with amino groups can be used. The solid substrate also contains at least one, preferable 2 independent fiducial marks.

Afterwards the m-RNA molecules are loaded on the surface with a buffer solution. The m-RNA molecules will interact with the functionalized surface and randomly spatially distribute on the entire area of the surface. The spatial or surface density of the m-RNA molecules can be controlled by the loaded concentration.

The spacer units and therefore in turn the m-RNA molecules are preferable randomly distributed on the substrate. The distribution density may be controlled via concentration, temperature, pH and surface functionalization. Preferable, the sample is permeabilized after providing to the surface.

### Step b)

A tissue sample is brought in contact with the solid substrate where all the single molecules are located. The 5-50 bases long poly T tail will then be hybridized to the expressed mRNA in the tissue.

### Step c)

The tissue can optionally be stained for example with DAPI or hematoxylin or eosin and imaged. Images are also taken of those fiducial marks and X-Y position are recorded. Each sequence of each individual single molecule is recorded and the spatial location relative to the fiducial marks is stored as X-Y distances.

After providing the sample to the surface, the sample may be stained to obtain the spatial location relative to the fiducial markers. Optionally, the sample is stained allowing to determine morphology of the sample.

### Step d)

The tissue is then removed from the substrate enzymatically or chemically, for example with proteinase K. Afterwards the single molecules with the mRNA on may then be reversely transcribed into cDNA.

As a next step the solid substrate with single molecules on it is denatured so that the double stranded DNA molecule is forming a single stranded oligonucleotide.

Optionally, the spatial location of the sample and the spatial location of the sequenced rolonies are superimposed relative to the location of the fiducial marker.

### Step e)

Padlock probes targeting various genes with a gap are added. The padlock probes will bind to the different mRNA if the gene is expressed. After that the gap is also reversely transcribed and the gap filled padlock are ligated into circle templates. Then rolling circle amplification is carried out on padlocks.

In a variant, the method of the invention is performed by hybridizing the at least one oligonucleotide to complementary parts of the at least one single stranded oligomer thereby creating a padlock unit with a gap between the 5' and the 3' end of the oligonucleotide and filling the gap of the padlock unit with complementary nucleic acids as target sequence and ligate them to generate the single strand circular template.

In another variant, the method of the invention is performed by hybridizing the at least one oligonucleotide to complementary parts of the at least one single stranded oligomer and ligate the 5' and the 3' end of the oligonucleotide to generate the single strand circular template, wherein the complementary parts of the at least one single stranded oligomer define the target sequence.

Preferable, the oligonucleotide comprises at least one primer sequence for the polymerase capable of rolling circle amplification. In alternative the oligonucleotide may be provided with at least one primer sequence for the polymerase capable of rolling circle amplification by ligation of a primer oligonucleotide.

In another variant, the oligonucleotide is provided with or comprises with at least two different primer sequences can be used which can be consecutively sequenced and help to avoid optical crowding if multiple rolonies light up next to each other.

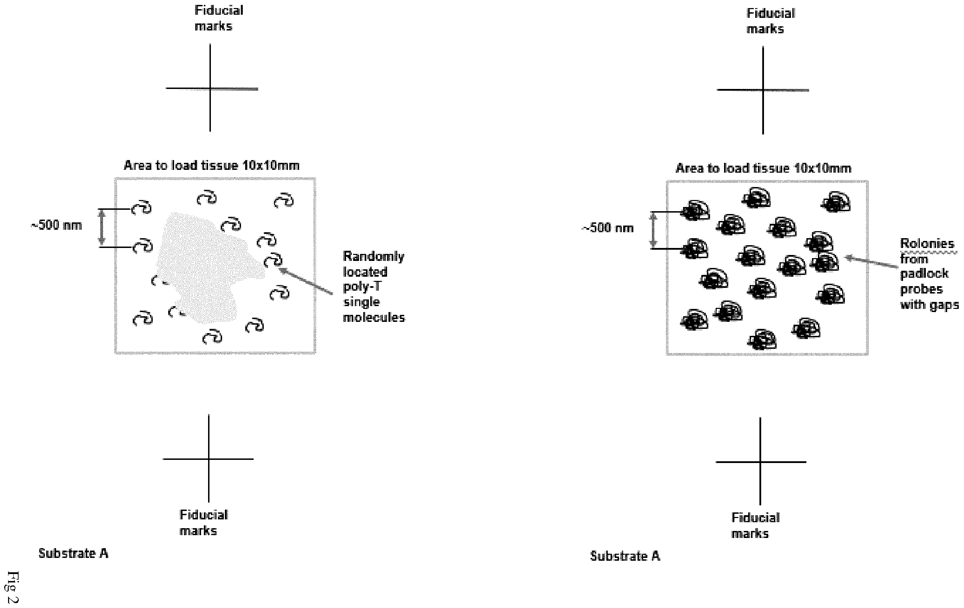

The location of each rolony on the substrate can be correlated with the original position of the tissue by taking one or more second images. Fig. 2 (A) shows taking the first image with tissue and fiducial markers; Fig. 2 (B) shows taking second images with the rolonies and fiducial markers during sequencing.

### Step f)

Finally, a sequencing primer is added and then the padlock with the filled gap is sequenced and each base is determined. The location of each rolony on the substrate can later on correlated with the original position of the tissue. That means the location of the gene on the tissue can be determined and it can be checked if the gene had a mutation or not via sequencing.

### Step g)

Preferable, sequencing information is obtained by sequencing by synthesis process. Sequencing by synthesis is performed by subsequently hybridizing fluorescently labelled nucleotides to the rollonies wherein the hybridized fluorescently labelled nucleotide provides a detectable fluorescence signal.

### Exemplary workflow based on variant with polyT tail as shown in Fig. 1:

- Load identical oligonucleotides (spacer units) consisting of a long polyT tail on to a functionalized solid surface (e.g. standard cover glass 25x75xlmm) (Fig. 1, A)
- Single molecules polyT will immobilize randomly on functionalized surface. Top view of the surface shown in Fig.2
- Density of single molecules on surface controlled via concentration
- The single molecules can have a minimal distance from each other of about 50-500 nm
- A tissue sample is brought in contact with the solid substrate where all the single molecules are located. The 30-50 bases long poly T tail will then be hybridized to the expressed mRNA in the tissue. (Fig. 1, B)
- The tissue will be stained ( DAPI/hematoxylin, eosin and imaged. The solid substrate also contains 2 independent fiducial marks. Images are also taken of those fiducial marks and X-Y position are recorded. Each sequence of each individual single molecule is recorded and the spatial location relative to the fiducial marks is stored as X-Y distances.
- An optional step would be to perform cyclic staining with different fluorochrome-conjugated antibodies to acquire microscopy data to further characterize the tissue sample and identify a few different protein expressed on the tissue. This gives more insights about the tissue and later on the protein information can be compared with the gene expression and variant results.
- The tissue is then removed from the substrate. Afterwards the single molecules with the mRNA on it are reversely transcribed into cDNA. (Fig. 1, C)
- As a next step the solid substrate with single molecules on it is denatured so that the double stranded DNA molecule is forming a single stranded oligonucleotide. (Fig. 1, D)
- Padlock probes targeting various genes with a gap are added. The padlock probes will bind to the different mRNA if the gene is expressed. (Fig. 1, E)
- After that the gap is also reversely transcribed and the gap filled padlock are ligated into circle templates. Then rolling circle amplification is carried out on padlocks. (Fig. 1, E)
- Finally, a sequencing primer is added and then the padlock with the filled gap is sequenced and each base is determined. The location of each rolony on the substrate can later on correlated with the original position of the tissue. That means the location of the gene on the tissue can be determined and it can be checked if the gene had a mutation or not via sequencing. (Fig. 1, F)

### Exemplary workflow based on variant with antibody capturing cap-end of mRNA as shown in Fig. 3

- Load identical antibody or aptamers on to a functionalized solid surface (e.g. standard cover glass 25x75xlmm) (Fig. 3, A)
- Antibody or aptamers will immobilize randomly on functionalized surface. Top view of the surface shown in Fig. 2
- Density of antibody or aptamers on surface controlled via concentration
- The antibody or aptamers can have a minimal distance from each other of about 50-500 nm
- A tissue sample is brought in contact with the solid substrate where all the antibody or aptamers are located and after permeabilization, the 7-methyl G will be recognized by the antibody or aptamer linked to the solid surface
- The tissue will be stained (DAPI/hematoxylin or eosin and imaged The solid substrate also contains 2 independent fiducial marks. Images are also taken of those fiducial marks and X-Y position are recorded. Each sequence of each individual single molecule is recorded and the spatial location relative to the fiducial marks is stored as X-Y distances.
- An optional step would be to perform cyclic staining with different fluorochrome-conjugated antibodies to acquire microscopy data to further characterize the tissue sample and identify a few different protein expressed on the tissue. This gives more insights about the tissue and later on, the protein information can be compared with the gene expression and variant results.
- The tissue is then removed from the substrate. (Fig. 3, B)
- Padlock probes targeting various genes with a gap are added. The padlock probes will bind to the different mRNA if the gene is expressed. (Fig. 3, C)
- After that the gap is also reversely transcribed and the gap filled padlock are ligated into circle templates. Then rolling circle amplification is carried out on padlocks. (Fig. 3, D)
- Finally a sequencing primer is added and then the padlock with the filled gap is sequenced and each base is determined. The location of each rolony on the substrate can later on correlated with the original position of the tissue. That means the location of the gene on the tissue can be determined and it can be checked if the gene had a mutation or not via sequencing. (Fig. 3, E)

## Claims

1. A method to obtain the spatial location and sequence information of a target sequence in a sample comprising at least one m-RNA strand comprising the steps
a. providing a surface with a plurality of spacer units capable of binding at least one m-RNA strand and with at least one fiducial marker
b. providing a sample comprising at least one m-RNA strand to the surface wherein at least one m-RNA strand of the sample binds to at least one spacer unit creating at least one single stranded oligomer
c. taking a first image of the surface to obtain the spatial information of the sample relative to the fiducial marker
d. removing sample form surface
e. hybridizing at least one oligonucleotide comprising 50 - 1000 nucleic acids with its 5' and 3' ends to complementary parts of the single stranded oligomer thereby forming a padlock-shaped structure that is ligated to create a single strand circular template
f. multiplying the single strand circular template by a polymerase capable of rolling circle amplification into a plurality of DNA concatemers thereby forming rolonies
g. obtaining the sequence information of the rolonies
h. linking the spatial information of the sample with the sequence information of the rolonies.

2. Method according to claim 1 **characterized in that** the spacer units are selected from the group consisting of antibodies, Fab fragments of antibodies, single chain Fv (scFv) fragments, divalent single chain antibodies or diabodies or aptamers .

3. Method according to claim 1 **characterized in that** the spacer units are selected from the group consisting of oligonucleotides comprising at least 5 Thymine (poly-T) single molecules and wherein the single stranded oligomer bond to the at least one spacer unit is reverse transcribed into a c-DNA strand and wherein the mRNA strand is removed by denaturation.

4. Method according to any of the claims 1 to 3 **characterized in that** hybridizing the at least one oligonucleotide to complementary parts of the at least one single stranded oligomer thereby creating a padlock unit with a gap between the 5' and the 3' end of the oligonucleotide and filling the gap of the padlock unit with complementary nucleic acids as target sequence and ligate them to generate the single strand circular template.

5. Method according to any of the claims 1 to 3 **characterized in that** hybridizing the at least one oligonucleotide to complementary parts of the at least one single stranded oligomer and ligate the 5' and the 3' end of the oligonucleotide to generate the single strand circular template, wherein the complementary parts of the at least one single stranded oligomer define the target sequence.

6. Method according to any of the claims 1 to 5 **characterized in that** the spacer units are randomly distributed on the substrate

7. Method according to any of the claims 1 to 6 **characterized in that** the sample is permeabilized after providing to the surface.

8. Method according to any of the claims 1 to 7 **characterized in that** in step d) the sample is removed form surface enzymatically or chemically.

9. Method according to any of the claims 1 to 8 **characterized in that** the oligonucleotide comprises at least one primer sequence for the polymerase capable of rolling circle amplification.

10. Method according to any of the claims 1 to 8 **characterized in that** the oligonucleotide is provided with at least one primer sequence for the polymerase capable of rolling circle amplification by ligation of a primer oligonucleotide.

11. Method according to claim 1 **characterized in that** the spatial location of the sample and the spatial location of the sequenced rolonies are superimposed relative to the location of the fiducial marker.

12. Method according to any of the claims 1 to 11 **characterized in that** after providing the sample to the surface, the sample is stained to obtain the spatial location relative to the fiducial markers.

13. Method according to any of the claims 1 to 12 **characterized in that** the sequencing information is obtained by sequencing by synthesis process

## Patentansprüche

1. Verfahren zum Erhalten der räumlichen Lage und Sequenzinformation einer Zielsequenz in einer Probe, die mindestens einen m-RNA-Strang umfasst, umfassend die Schritte
a. Bereitstellen einer Oberfläche mit einer Vielzahl von Spacer-Einheiten, die fähig sind, mindestens einen m-RNA-Strang zu binden, und mit mindestens einem Referenzmarker,
b. Bereitstellen einer Probe, die mindestens einen m-RNA-Strang umfasst, auf der Oberfläche, wobei mindestens ein m-RNA-Strang der Probe an mindestens eine Spacer-Einheit bindet und mindestens ein einzelsträngiges Oligomer erzeugt,
c. Aufnehmen eines ersten Bildes der Oberfläche, um die räumliche Information der Probe in Bezug zum Referenzmarker zu erhalten,
d. Entfernen der Probe von der Oberfläche,
e. Hybridisieren mindestens eines Oligonukleotids, das 50 bis 1.000 Nukleinsäuren umfasst, mit seinen 5'- und 3'-Enden an komplementäre Teile des einzelsträngigen Oligomers, wodurch eine Padlock-förmige Struktur gebildet wird, die ligiert wird, um eine einzelsträngige zirkuläre Matrize zu erzeugen,
f. Vervielfältigen der einzelsträngigen zirkulären Matrize durch eine Polymerase, die zur Rolling-Circle-Amplifikation fähig ist, in eine Vielzahl von DNA-Concatemeren, wodurch Rolonies gebildet werden,
g. Erhalten der Sequenzinformation der Rolonies"
h. Verknüpfen der räumlichen Information der Probe mit der Sequenzinformation der Rolonies".

2. Verfahren nach Anspruch 1, **dadurch gekennzeichnet, dass** die Spacer-Einheiten ausgewählt sind aus der Gruppe bestehend aus Antikörpern, Fab-Fragmenten von Antikörpern, einzelkettigen Fv-Fragmenten (scFv), zweiwertigen einzelkettigen Antikörpern oder Diabodies oder Aptameren.

3. Verfahren nach Anspruch 1, **dadurch gekennzeichnet, dass** die Spacer-Einheiten ausgewählt sind aus der Gruppe, bestehend aus Oligonukleotiden, die mindestens 5 Thymin-Einzelmoleküle (poly-T) umfassen, und wobei die einzelsträngige Oligomerbindung an die mindestens eine Spacer-Einheit in einen c-DNA-Strang revers transkribiert wird und wobei der mRNA-Strang durch Denaturierung entfernt wird.

4. Verfahren nach einem der Ansprüche 1 bis 3, **dadurch gekennzeichnet, dass** das mindestens eine Oligonukleotid an komplementäre Teile des mindestens einen einzelsträngigen Oligomers hybridisiert wird, wodurch eine Padlock-Einheit mit einer Lücke zwischen dem 5'- und dem 3'-Ende des Oligonukleotids erzeugt wird, und die Lücke der Padlock-Einheit mit komplementären Nukleinsäuren als Zielsequenz gefüllt wird und diese ligiert werden, um die einzelsträngige zirkuläre Matrize zu erzeugen.

5. Verfahren nach einem der Ansprüche 1 bis 3, **dadurch gekennzeichnet, dass** das mindestens eine Oligonukleotid an komplementäre Teile des mindestens einen einzelsträngigen Oligomers hybridisiert und das 5'- und das 3'-Ende des Oligonukleotids ligiert werden, um die einzelsträngige zirkuläre Matrize zu erzeugen, wobei die komplementären Teile des mindestens einen einzelsträngigen Oligomers die Zielsequenz definieren.

6. Verfahren nach einem der Ansprüche 1 bis 5, **dadurch gekennzeichnet, dass** die Spacer-Einheiten zufällig auf dem Substrat verteilt sind.

7. Verfahren nach einem der Ansprüche 1 bis 6, **dadurch gekennzeichnet, dass** die Probe nach dem Bereitstellen auf die Oberfläche permeabilisiert wird.

8. Verfahren nach einem der Ansprüche 1 bis 7, **dadurch gekennzeichnet, dass** in Schritt d) die Probe enzymatisch oder chemisch von der Oberfläche entfernt wird.

9. Verfahren nach einem der Ansprüche 1 bis 8, **dadurch gekennzeichnet, dass** das Oligonukleotid mindestens eine Primersequenz für die zur Rolling-Circle-Amplifikation fähige Polymerase umfasst.

10. Verfahren nach einem der Ansprüche 1 bis 8, **dadurch gekennzeichnet, dass** das Oligonukleotid mit mindestens einer Primer-Sequenz für die zur Rolling-Circle-Amplifikation fähige Polymerase durch Ligation eines Primer-Oligonukleotids versehen ist.

11. Verfahren nach Anspruch 1, **dadurch gekennzeichnet, dass** die räumliche Lage der Probe und die räumliche Lage der sequenzierten Rolonies" relativ zur Lage des Referenzmarkers übereinander gelegt werden.

12. Verfahren nach einem der Ansprüche 1 bis 11, **dadurch gekennzeichnet, dass** nach dem Bereitstellen der Probe auf der Oberfläche die Probe angefärbt wird, um die räumliche Lage in Bezug zu den Referenzmarkern zu erhalten.

13. Verfahren nach einem der Ansprüche 1 bis 12, **dadurch gekennzeichnet, dass** die Sequenzierungsinformation durch Sequenzierung mittels Syntheseverfahren erhalten wird.

## Revendications

1. Procédé d'obtention de la localisation spatiale et des informations de séquence d'une séquence cible dans un échantillon comprenant au moins un brin d'ARNm comprenant les étapes consistant à
a. fournir une surface avec une pluralité d'unités d 'espaceur capables de se lier à un brin d'ARNm et avec au moins un marqueur fiduciaire
b. fournir un échantillon comprenant au moins un brin d'ARNm à la surface au moins un brin d'ARNm se liant à au moins une unité d'espaceur créant au moins un oligomère simple brin
c. prendre une première image de la surface pour obtenir les informations spatiales de l'échantillon par rapport au marqueur fiduciaire
d. enlever l'échantillon de la surface
e. hybrider au moins un oligonucléotide comprenant 50 à 1000 acides nucléiques avec ses extrémités 5' et 3' aux parties complémentaires de l'oligomère simple brin formant ainsi une structure en forme de cadenas qui est liguée pour créer une matrice circulaire simple brin
f. multiplier la matrice circulaire simple brin par une polymérase capable d'amplification en cercle roulant en une pluralité de concatémères d'ADN formant ainsi des rolonies
g. obtenir les informations de séquence des rolonies
h. lier les informations spatiales del 'échantillon avec les informations de séquence des rolonies.

2. Procédé selon la revendication 1 **caractérisé en ce que** les unités d'espaceur sont choisies dans le groupe constitué par les anticorps, les fragments Fab d'anticorps, les fragments monocatéaires de Fv (scFv), les anticorps monocaténaires divalents ou les diabodies ou les aptamères.

3. Procédé selon la revendication 1 **caractérisé en ce que** les unités d'espaceur sont choisies dans le groupe constitué par les oligonucléotides comprenant au moins des simples molécules de 5 thymine (poly-T) et la liaison de l'oligomère simple brin à l'au moins une unité d'espaceur étant transcrite en inverse en un brin d'ADNc et le brin d'ARNm étant enlevé par dénaturation.

4. Procédé selon l'une quelconque des revendications 1 à 3 **caractérisé en ce que** l'hybridation de l'au moins un oligonucléotide aux parties complémentaires de l'au moins un oligomère simple brin créant ainsi une unité de cadenas avec un espace libre entre les extrémités 5' et 3' de l'oligonucléotide et le remplissage de l'espace libre de l'unité de cadenas avec les acides nucléiques complémentaires comme séquence cible et leur ligation pour produire la matrice circulaire simple brin.

5. Procédé selon l'une quelconque des revendications 1 à 3 **caractérisé en ce que** l'hybridation de l'au moins un oligonucléotide aux parties complémentaires de l'au moins un oligomère simple brin et la ligation des extrémités 5' et 3' de l'oligonucléotide pour produire la matrice circulaire simple brin, les parties complémentaires de l'au moins un oligomère simple brin définissant la séquence cible.

6. Procédé selon l'une quelconque des revendications 1 à 5 **caractérisé en ce que** les unités d'espaceur sont distribuées au hasard sur le substrat.

7. Procédé selon l'une quelconque des revendications 1 à 6 **caractérisé en ce que** l'échantillon est perméabilisé après avoir été fourni à la surface.

8. Procédé selon l'une quelconque des revendications 1 à 7 **caractérisé en ce que** dans l'étape d) l'échantillon est enlevé de la surface enzymatiquement ou chimiquement.

9. Procédé selon l'une quelconque des revendications 1 à 8 **caractérisé en ce que** l'oligonucléotide comprend au moins une séquence d'amorce pour la polymérase capable d'amplification en cercle roulant.

10. Procédé selon l'une quelconque des revendications 1 à 8 **caractérisé en ce que** l'oligonucléotide est fourni avec au moins une séquence d'amorce pour la polymérase capable d'amplification en cercle roulant par ligation d'un oligonucléotide amorce.

11. Procédé selon la revendication 1 **caractérisé en ce que** la localisation spatiale de l'échantillon et la localisation spatiale des rolonies séquencées sont surimposées par rapport à la localisation du marqueur fiduciaire.

12. Procédé selon l'une quelconque es revendications 1 à 11 **caractérisé en ce qu'**après la fourniture de l'échantillon à la surface, l'échantillon est coloré pour obtenir la localisation spatiale par rapport aux marqueurs fiduciaires.

13. Procédé selon l'une quelconque des revendications 1 à 12 **caractérisé en ce que** les informations de séquençage sont obtenues par séquençage par un processus de synthèse.
